(19) Europäisches Patentamt
European Patent Office
Office européen des brevets

(11) Veröffentlichungsnummer: **0 164 655 B1**

# (12) EUROPÄISCHE PATENTSCHRIFT

(45) Veröffentlichungstag der Patentschrift: **29.05.91**

(51) Int. Cl.5: **C12P 19/18**, C12P 19/08, A61K 31/70

(21) Anmeldenummer: **85106656.3**

(22) Anmeldetag: **30.05.85**

Die Akte enthält technische Angaben, die nach dem Eingang der Anmeldung eingereicht wurden und die nicht in dieser Patentschrift enthalten sind.

## (54) Verfahren zur Herstellung monovalenter Haptene.

(30) Priorität: **15.06.84 DE 3422246**

(43) Veröffentlichungstag der Anmeldung: **18.12.85 Patentblatt 85/51**

(45) Bekanntmachung des Hinweises auf die Patenterteilung: **29.05.91 Patentblatt 91/22**

(84) Benannte Vertragsstaaten: **AT BE CH DE FR GB LI NL**

(56) Entgegenhaltungen:
**EP-A- 0 061 738**
**AU-B- 519 216**
**DE-A- 2 630 596**

**CARBOHYDRATE RESEARCH, Band 108, 1982, Seiten 279-283; Elsevier Scientific Publ. Co., Amsterdam, NL; K.L. SMILEY et al.:"A simplified method for preparting linear isomalto-oligosaccharides"**

**CHEMICAL ABSTRACTS, Band 90, 1979, Seiten 413-414, Ref.Nr. 166270w; Columbus, Ohio, US; J.C. JATON et al.:"Interactions of monovalent and multivalent oligosaccharide ligands with homogeneous anti-polysaccharide antibody and its possible effects on the conformation of the IgG molecule"**

(73) Patentinhaber: **PFEIFER & LANGEN**
**Linnicher Strasse 48**
**W-5000 Köln 41(DE)**

(72) Erfinder: **Schwengers, Dieter, Dr.**
**Jussenhovener Strasse 37**
**W-4047 Dormagen 1(DE)**

(74) Vertreter: **Eggert, Hans-Gunther, Dr.**
**Räderscheidtstrasse 1**
**W-5000 Köln 41(DE)**

EP 0 164 655 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Seit langem wird klinisches Dextran als kolloidales Volumenersatzmittel bei der Behandlung des Volumenmangelschocks und zur Thromboseprophylaxe in großen Mengen eingesetzt.

Es ist bekannt, daß die Inzidenz unerwünschter Dextraninduzierter anaphylactoider Nebenwirkungen (DIAR) durch iso-Malto-Oligosaccharide verhindert werden kann. Als besonders wirksam erwiesen sich iso-Malto-pentaose (IM-5), iso-Malto-Hexaose (IM-6) und iso-Maltoheptaose (IM-7). Während iso-Malto-tetraose (I-4) deutlich weniger effektiv ist und iso-Maltotetraose (IM-3) kaum noch wirksam ist, steigt mit wachsender Kettenlänge die Tendenz zur Präzipitatsbildung. Iso-Maltododekaose (IM-12) führt in vitro wieder zur Bildung von Präzipitaten. IM-5 bis IM-7 stellen antigene Determinanten des Dextrans dar, weil sie sehr stark von Dextran-Antikörpern gebunden werden, ohne daß es zu einer Präzipitatsbildung kommt. IM-5 bis IM-7 wirken demnach als monovalente Haptene, während IM-12 bereits wieder ein bivalentes Antigen darstellt.

Gemische von iso-Malto-Oligosaccahariden, die eine geeignete Zusammensetzung als Hapten zur Prophylaxe von DIAR aufweisen, werden durch Säurehydrolyse von klinischem Dextran oder Dextranfraktionen eines mittleren Molekulargewichts von 17000 und nachfolgende Fällungsfraktionierung mit Ethanol gewonnen. Die klinischen Dextrane werden ihrerseits durch partielle Säurehydrolyse aus hochmolekularen nativen Dextran mit spezieller Steuerung der Hydrolyse-Bedingungenund durch eine sorgfältige Fällungsfraktionierung mit Lösungsmitteln hergestellt.

Die bekannten Verfahren zur Herstellung monovalenter Haptene haben zunächst den Nachteil, daß sie von partiell hydrolysiertem fraktioniertem und damit teurem Dextran ausgehen. Für die Gewinnung von 25 kg der iso-Malto-Oligosaccharide IM-5 bis IM-7 auf dem Umweg über natives und klinisches Dextran, werden etwa 1.000 kg Saccharose benötigt. Hinzu kommen die kostenintensiven Fraktionierungsmaßnahmen. Zur Gewinnung der (klinischen) Dextrane eines bestimmten vorgegebenen mittleren Molekulargewichts.

Damit die Oligosaccaridgemisch-Aubeute bezogen auf eingesetztes Dextran nicht auf ein wirtschaftlich nicht zu vertretendes Maß absinkt, werden bei der Fraktionierung Glucose, iso-Maltose und iso-Maltotriose, die keine Haptenwirkung gegen Dextranantikörper haben, und iso-Malto-Oligosaccaride höheren Polymerisationsgrades als 11, die bereits wieder als bivalente Antigene die Präzipitatsbildung fördern, im Gemisch belassen.

So besteht ein im Handel erhältliches iso-Malto-Oligosaccardidgemisch zu ca. 20 % aus den unwirksamen Komponenten Glucose, iso-Maltose und isoMaltotriose und zu ca. 10 % aus iso-Malto-Oligosacchariden höheren Polymerisationsgrades als 11.

Da das iso-Malto-Oligosaccaridgemisch durch Säurehydrolyse von Dextran hergestellt wird, das einen, wenn auch geringen Verzweigungsgrad aufweist, sind auch die Oligosaccaride des Gemisches in geringem Umfang verzweigt. Ihr Anteil leistet keinen Beitrag zur Haptenwirkung des Gemisches.

Wegen dieser Nachteile hat die Verabreichung der nach dem bekannten Verfahren hergestellten iso-Malto-Oligosaccaride als Vorinjektion zu klinischem Dextran zur Verhinderung von DIAR nicht voll befriedigt.

AU-B-1-36 758/78 beschreibt ein iso-Malto-Oligosaccharidgemisch. Dieses wurde durch Dextranhydrolyse, Isolierung der iso-Malto-Oligosaccharide und Zusammenfügen der einzelnen iso-Malto-Oligosaccharide gemäß dieser Druckschrift hergestellt.

Aus Carbohydrate Research, 108, (1982), 279-283, ist ein Verfahren zur Bereitstellung von einzelnen iso-Malto-Oligosacchariden als Vergleichsverbindungen für die Flüssigkeitschromatographie bekannt. Danach erfolgt ein Aufbau von iso-Malto-Oligosacchariden in einer Reaktionsmischung aus 0,3 M Saccharose und 0,4 M D-Glucose in Gegenwart von 1400 U Dextransucrase bei einem pH-Wert von 5,0 und einer Temperatur von 4 °C. Nach 24 Stunden wird die Reaktion durch Methanolzugabe beendet und in weiteren Verfahrensschritten die einzelnen iso-Malto-Oligosaccharide für den Einsatz als Referenzsubstanzen isoliert.

Der Erfindung liegt die Aufgabe zugrunde, monovalente Haptene zur Prophylaxe von DIAR herzustellen, die möglichst nur unverzweigte iso-Malto-Oligosaccharide bei einem hohen Anteil von IM-4, IM-5, IM-6 und IM-7 enthalten.

Diese Aufgabe wird durch das erfindungsgemäße Verfahren gelöst, welches sich dadurch auszeichnet, daß man einer wässrigen Lösung von D-Glucose, die mehr als 300 mMol Glucose pro 1000 U $\alpha(1\rightarrow6)$-D-Glucosyltransferase enthält, bei 265 bis 310 K und einem pH-Wert von 4,5 bis 8,0 eine wässrige Saccharoselösung in einer solchen Menge zugibt, daß das Molverhältnis von Saccharose zu Glucose 0,5 bis 2,0 beträgt, und nach Verbrauch der Saccharose Glucose, freigesetzte Fructose und unerwünschte Oligosaccharide in an sich bekannter Weise abtrennt.

Das Reaktionsgemisch wird vorzugsweise bei 290 bis 300 K und in einem pH-Wert-Bereich von 5 bis 6,5 gehalten. Beide Parameter haben einen Einfluß auf die Struktur der entstehenden Produkte.

Als $\alpha(1\rightarrow6)$-D-Glucosyltransferase nach der Klassifizierung der "Enzyme Commission" bezeichnet man

Enzyme, die die D-Glucopyranosylgruppe der Saccharose auf geeignete Akzeptoren übertragen. Ein solches extracellulares Enzym ist Dextransucrase (E.C. 2.4.1.5) das von gewissen Bakterienarten der Lactobacilli- Familie, z.B. Leuconostoc mesenteroides, insbesondere dem Stamm B-512, Leuconostoc dextranicum, Streptococcus und Lactobacillus gebildet wird. Zur Herstellung von Dextran dient in erster Linie Saccharose als Akzeptor und wirkt als Ketteninitiator für eine Kettenpolymerisation, bei der durch fortlaufende Übertragung von D-Glucopyranosyl-Gruppen aus der Saccharose auf die wachsende Kette das Polysaccharid Dextran mit Molmassen von mehreren Millionen gebildet wird, während gleichzeitig für jedes Molekül umgesetzter Saccharose ein Molekül Fructose frei wird.

Wenn man bei dieser Reaktion andere Mono-, Di- oder Tri-saccharide als Akzeptor einsetzt, werden auf Kosten des Dextrans in geringem Umfang Oligosaccharide gebildet. Bei Einsatz von Glucose als Akzeptor entstehen etwa 78 % Dextran und als Nebenprodukt etwa 9 % Oligosaccharide (IM-3 bis IM-12). (Robyt und Eklund, Carbohydrate Research 121(1983), 279-286). Typischerweise werden die Oligosaccharide mit steigendem Polymerisationsgrad in abnehmender Menge gebildet.

Unter den erfindungsgemäßen Reaktionsbedingungen gelingt es, die Übertragung von Glucosyl-Gruppen aus der Saccharose auf Glucose so zu lenken, daß kein Dextran mehr entsteht, sondern in hoher Ausbeute die iso-Malto-Oligosaccharide IM-4 bis IM-8 aufgebaut werden. Überraschend dabei ist, daß die Oligosaccharide mit steigendem Polymerisationsgrad nicht mehr in abnehmenderMenge gebildet werden, sondern daß ein Häufigkeitsmaximum bei IM-4 bis IM-6 vorliegt.

Bei dem erfindungsgemäßen Verfahren empfiehlt es sich zur Erzielung hoher Ausbeuten der gewünschten Haptene, die wässrige Lösung von Saccharose mit einer solchen Geschwindigkeit kontinuierlich zuzugeben, daß die vorgelegte Enzymmenge die zulaufende Saccharosemenge sofort umsetzen kann und so eine Anreicherung von Saccharose im Reaktionsgemisch, die zur unkontrollierten Bildung von hochmolekularem Dextran führen kann, vermieden wird. In jedem Fall sollte der Anteil der Saccharose an der Kohlehydrat- Trockensubstanz des Reaktionsgemisches im Gleichgewichtszustand der kontinuierlichen Umsetzung 10 % nicht überschreiten.

Anstelle der gereinigten Dextransucrase kann auch das Gemisch aus dem Enzym und den es produzierenden Bakterien eingesetzt werden.

Die Aufbaureaktion läßt sich wie folgt darstellen:

$$\text{Glucose + n Saccharose} \xrightarrow{\text{Dextransucrase}} \text{iso-Malto-(n+1)-Saccharide + n Fructose}$$

In dieser Gleichung ist n die Anzahl der Mole eingesetzter Saccharose.

Diese Reaktion läßt sich erfindungsgemäß so steuern, daß iso-Malto- Oligo- oder Polysaccharide des jeweils gewünschten Molekulargewichts erhalten werden. Unter den genannten Bedingungen der Temperatur und der Wasserstoffionenkonzentration hängt das bei dieser Aufbausynthese erreichte Molekulargewicht von der auf eine bestimmte Enzymaktivität der vorgelegten Lösung bezogenen molaren Menge des Akzeptors (Glucose) einerseits und dem Molverhältnis der insgesamt zugeführten Saccharose zum Akzeptor andererseits ab.

Die Enzymmenge, die 1 $\mu$Mol Saccharose pro min bei pH 5,2 und 298 K umsetzt, ist die Enzymaktivitätseinheit = Unit = U. Wenn mehr Saccharose zugeführt wird, als die vorgelegte Enzym-Aktivität umsetzen kann, ist die Steuerung der Molekülgröße nicht mehr möglich. Bezogen auf den gleichen Saccharose-Umsatz erhöht sich das erreichte Molekulargewicht mit kleiner werdendem Molverhältnis von Glucose zur Enzym-Aktivität.

Legt man als Enzym-aktivität 1000 U zugrunde, so wird bei einem Saccharoseumsatz von insgesamt 1000 mMol und 400 - 800 mMol, z.B. 600 mMol Glucose das gewünschte Oligo-Saccharidgemisch mit einem mittleren Molekulargewicht von etwa 1000 bis 1200 erhalten. Das Molverhältnis der Saccharose zur vorgelegten Glucose beträgt 0,5 - 2,0 : 1, vorzugsweise 0,8 - 1,2 : 1, insbesondere 1 : 1.

Es ist also möglich, in wenigen Vorversuchen mit wechselnden molaren Mengen an Glucose innerhalb des aufgezeigten Bereichs bei vorgegebener Aktivität der $\alpha(1\rightarrow6)$-D-Glucosyltransferase (z.B. 1000 U) und einer konstanten Menge Saccharose (z.B. 1000 mMol) die Anlagerung der D-Glucopyranosyl-Gruppen der Saccharose an die Glucose als Akzeptor so zu steuern, daß in hoher Ausbeute Fraktionen der jeweils gewünschten iso-Malto-Oligosaccharide mit enger Molekulargewichtsverteilung synthetisiert werden können.

Man kann die gesamte erforderliche Glucosemenge vorlegen oder unter Wahrung der übrigen Reaktionsbedingungen, insbesondere der Konzentrationsverhältnisse auch die Glucose kontinuierlich in dem Maße ersetzen, wie sie als Akzeptor verbraucht wird. Es ist also möglich, die Synthesereaktion voll

kontinuierlich durchzuführen.

Es ist ein unerwarteter Vorteil der erfindungsgemäßen Verfahrensweise, daß der Kohlehydrat-Trockensubstanz-Gehalt des Reaktionsgemisches sehr hoch sein kann, indem er 30 bis 50 %, insbesondere 40 bis 50 % beträgt. Dabei hat sich überraschenderweise gezeigt, daß die Bildung des Disaccharids Leucrose, verglichen mit einer chargenweise Durchführung der enzymatischen Reaktion erheblich reduziert wird.

Ein weiterer überraschender Vorteil des erfindungsgemässen Verfahrens ist darin zu sehen, daß die nach diesem Verfahren hergestellten iso-Malto-Oligosaccharide weniger durch verzweigte Oligosaccharide verunreinigt sind als dies bei den durch Säurehydrolyse von Dextranen gewonnenen der Fall ist.

Es hat sich gezeigt, daß bei der kontinuierlichen Zufuhr der Saccharose der Anteil an iso-Malto-Oligosacchariden mit 10 oder mehr Anhydroglucose-Einheiten (≧ IM-10) ein brauchbarer Indikator für die Bildung eines hohen Anteils der gewünschten Monovalenten Haptene IM-5 bis IM-7 ist. Dementsprechend empfiehlt es sich, die Saccharosezufuhr zu beenden, wenn der Anteil an IM = 10 etwa 10 % der Kohlehydrat-Trockensubstanz des Reaktionsgemisches erreicht hat.

Der Anteil an IM-4 bis IM-8 der Kohlehydrat-Trockensubstanz des Reaktionsgemisches beträgt dann etwa 25 %.

Obgleich bei der erfindungsgemäßen enzymatischen Aufbausynthese steril gearbeitet wird, wie das z.B. für die Synthese von nativem Dextran üblich ist, können dem Reaktionsgemisch zur Vermeidung von unerwünschtem Hefewachstum Antimitotika (Mytosehemmer), wie schweflige Säure in Mengen bis zu 1000 mg/kg, insbesondere 400 bis 600 mg/kg zugesetzt werden.

Die Gewinnung der gewünschten monovalenten Haptene aus dem Reaktionsgemisch durch Abtrennung der nicht umgesetzten Glucose, der freigesetzten Fructose, der Di- und Trisaccharide und der iso-Malto-Oligosaccharide mit einem höheren Polymerisationsgrad als 11, kann nach an sich bekannten Methoden, z.B. durch Fällungsfraktionierung mit Ethanol, erfolgen. Als sehr geeignet hat sich die Abtrennung der Nebenprodukte durch Chromatographie über eine mit einem stark sauren Kationenaustauscher gefüllte Säure erwiesen.

Das erfindungsgemäße Verfahren hat nicht nur den oben beschriebenen Vorteil der Bildung unverzweigter iso-Malto-Oligosaccharide bei hohem Anteil an IM-4 bis IM-8, insbesondere IM-5 bis IM-7, die sich als monovalente Haptene bestimmungsgemäß besonders gut zur Verhinderung anaphylactoider Nebenwirkungen des Dextrans eignen, sondern es bedeutet auch verglichen mit dem Umweg über natives und klinisches Dextran die Reduzierung der für die gleiche Menge Hapten benötigten Menge an Saccharose auf etwa ein Zehntel, d.h. für 25 kg Hapten werden statt 1000 kg Saccharose nur noch 100 kg benötigt.

Das nach dem erfindungsgemäßen Verfahren erhaltene Reaktionsgemisch aus den iso-Malto-Oligosacchariden einschließlich der Mono- und Disaccharide kann direkt als kalorienarmes Süßungsmittel verwendet werden. Nach Abtrennung der Mono- und Disaccharide kann das verbleibende iso-Malto-Oligosaccharidgemisch als fast kalorienfreier Träger für Süßstoffe verwendet werden.

## Beispiel

In 30 l einer wässrigen Lösung des Enzyms Dextransucrase, die eine Aktivität von 5.800 U/l hatte, wurden 22 kg kristalline Glucose bei 298 K gelöst. der pH-Wert der Lösung betrug 5,4. Zu dieser Lösung wurden kontinuierlich 100 kg einer 40 %-Saccharoselösung mit einem pH-Wert von 5,4 innerhalb von 24 h zugepumpt. 2 Stunden nach beendeter Saccharosezugabe wurde das Enzym durch Erwärmen des Reaktionsgemisches auf 70 °C inaktiviert.

Die Analyse des Saccharidgemisches ergab:

| | | | |
|---|---|---|---|
| >IM-10 | 1,3 | | |
| IM-10 | 1,2 | IM-4 | 6,2 |
| IM-9 | 2,6 | IM-3 | 4,1 |
| IM-8 | 3,1 | IM-2 | 4,6 |
| IM-7 | 4,1 | Leucrose | 4,0 |
| IM-6 | 5,3 | Glucose | 25,8 |
| IM-5 | 7,4 | Fructose | 30,3 |

51 Liter dieser Saccharidlösung wurden auf eine chromatografische Trennanlage gegeben, die 400 l stark saures, mit Calciumionen beladenes Kationenaustauscherharz enthielt und die einzelnen Saccharide wurden durch Zuführen von 43 l/h destilliertem Wasser aus der Säule eluiert.

Nach einem Vorlauf von 54 l wurde die Hauptmenge der iso-Malto-Oligosaccharide innerhalb der nächsten 35 l aus der Trennsäule eluiert. Diese Fraktion hatte folgende Zusammensetzung

| | | | |
|---|---|---|---|
| >IM-10 | 6,6 | IM-6 | 15,9 |
| IM-10 | 6,1 | IM-5 | 19,9 |
| IM-9 | 7,8 | IM-4 | 13,7 |
| IM-8 | 10,6 | IM-3 | 4,9 |
| IM-7 | 13,5 | IM-2 | 1,0 |

Sie bestand demnach zu 63 % aus den besonders erwünschten Sacchariden IM-7, IM-6, IM-5 und IM-4.

Aus diesem Gemisch wurde IM-5 durch Gelchromatographie isoliert und seine vollständig lineare Struktur durch Kernresonanzspektroskopie bestätigt.

## Ansprüche

1. Verfahren zur Herstellung monovalenter Haptene, dadurch gekennzeichnet, daß man einer wässrigen Lösung von D-Glucose, die 400 bis 800 mmol Glucose pro 1000 U$\alpha$(1→6)-D-Glucosyltransferase enthält, bei 265 bis 310 K und einem pH-Wert von 4,5 bis 8,0 eine wässrige Saccharoselösung in einer solchen Menge zugibt, daß das Molverhältnis von Saccharose zu Glucose 0,5 bis 2,0 beträgt, und nach Verbrauch der Saccharose Glucose, freigesetzte Fructose und unerwünschte Oligosaccharide in an sich bekannter Weise abtrennt.

2. Verfahren nach Anspruch 1, dadurch gekennzeichnet, daß die Enzymreaktion bei 290 bis 300 K durchgeführt wird.

3. Verfahren nach Anspruch 1 oder 2, dadurch gekennzeichnet, daß der pH-Wert des Reaktionsgemisches 5 bis 6,5 beträgt.

4. Verfahren nach einem der Ansprüche 1 bis 3, dadurch gekennzeichnet, daß das Molverhältnis von Saccharose zu Glucose 0,8 bis 1,2, insbesondere 1 : 1 beträgt.

5. Verfahren nach einem der Ansprüche 1 bis 4, dadurch gekennzeichnet, daß man die Saccharoselösung kontinuierlich zugibt.

6. Verfahren nach einem der Ansprüche 1 bis 5, dadurch gekennzeichnet, daß die Saccharoselösung mit einer solchen Geschwindigkeit zugegeben wird, daß die Saccharose direkt von der $\alpha$(1→6)-D-Glucosyltransferase umgesetzt wird.

7. Verfahren nach einem der Ansprüche 1 bis 6, dadurch gekennzeichnet, daß die Saccharosezufuhr beendet wird, wenn der Anteil an iso-Malto-Oligosacchariden mit 10 oder mehr Anhydroglucoseeinheiten 10 % der Kohlehydrattrockensubstanz des Reaktionsgemisches erreicht hat.

8. Verfahren nach einem der Ansprüche 1 bis 7, dadurch gekennzeichnet, daß der Kohlehydrattrockensubstanzgehalt des Reaktionsgemisches 30 bis 50 %, insbesondere 40 bis 50 %, beträgt.

9. Verfahren nach einem der Ansprüche 1 bis 8, dadurch gekennzeichnet, daß man als $\alpha$(1→6)-D-Glucosyltransferase die vom Bakterium Leuconostoc mesenteroides, insbesondere dem Stamm B-512, ausgeschiedene Dextransucrase verwendet.

10. Verfahren nach einem der Ansprüche 1 bis 8, dadurch gekennzeichnet, daß man als α(1→6)-D-Glucosyltransferase die vom Bakterium Leuconostoc dextranicum ausgeschiedene Dextransucrase verwendet.

11. Verfahren nach einem der Ansprüche 1 bis 10, dadurch gekennzeichnet, daß man die Abtrennung der Nebenprodukte durch Chromatographie über eine mit einem stark sauren Kationenaustauscher gefüllte Säule vornimmt.

12. Verfahren nach einem der Ansprüche 1 bis 11, dadurch gekennzeichnet, daß man die Nebenprodukte durch Fällungsfraktionierung mit Lösungsmitteln entfernt.

13. Verfahren nach einem der Ansprüche 1 bis 12, dadurch gekennzeichnet, daß man die D-Glucose kontinuierlich in dem Maße ersetzt, wie sie als Akzeptor verbraucht wird.

14. Verfahren nach einem der Ansprüche 1 bis 13, dadurch gekennzeichnet, daß man dem Reaktionsgemisch zur Vermeidung von unerwünschtem Hefewachstum Antimitotika (Mytosehemmer) zusetzt.

15. Verfahren nach Anspruch 14, dadurch gekennzeichnet, daß man schwefelige Säure in Mengen bis zu 1000 mg/kg, vorzugsweise 400 bis 600 mg/kg, zusetzt.

16. Verwendung eines nach einem der Ansprüche 1 bis 15 erhaltenen Gemisches der vollständig linearen iso-Malto-Oligosaccharide IM-4 bis IM-8, insbesondere IM-5 bis IM-7, zur Herstellung eines Arzneimittels zur Verhinderung anaphylaktoider Nebenwirkungen des Dextrans.

## Claims

1. Method for preparing monovalent haptenes, characterised in that an aqueous sucrose solution is added to an aqueous solution of D-glucose which contains 400 to 800 mmol glucose per 1000 U α(1→6)-D-glucosyltransferase at 265 to 310 K and a pH value of 4.5 to 8.0 in such a quantity that the molar ratio of sucrose to glucose is 0.5 to 2.0, and once the sucrose has been used up glucose, released fructose and unwanted oligosaccharides are separated off in known manner.

2. Method according to Claim 1, characterised in that the enzyme reaction is carried out at 290 to 300 K.

3. Method according to Claim 1 or 2, characterised in that the pH value of the reaction mixture is 5 to 6.5.

4. Method according to one of Claims 1 to 3, characterised in that the molar ratio of sucrose to glucose is 0.8 to 1.2, in particular 1 : 1.

5. Method according to one of Claims 1 to 4, characterised in that the sucrose solution is added continuously.

6. Method according to one of Claims 1 to 5, characterised in that the sucrose solution is added at such a rate that the sucrose is reacted directly from the α(1→6)-D-glucosyltransferase.

7. Method according to one of Claims 1 to 6, characterised in that the supply of sucrose is terminated when the proportion of iso-malto-oligosaccharides with 10 or more anhydroglucose units has reached 10% of the carbohydrate dry substance of the reaction mixture.

8. Method according to one of Claims 1 to 7, characterised in that the carbohydrate dry substance content of the reaction mixture is 30 to 50%, in particular 40 to 50%.

9. Method according to one of Claims 1 to 8, characterised in that the dextransucrase separated from the bacterium leuconostoc mesenteroides, in particular the B-512 strain, is used as the α(1→6)-D-glucosyltransferase.

10. Method according to one of Claims 1 to 8, characterised in that the dextransucrase separated from the

bacterium leuconostoc dextranicum is used as the α(1→6)-D-glucosyltransferase.

11. Method according to one of Claims 1 to 10, characterised in that the separation of the byproducts is performed by chromatography over a column filled with a strongly acid cation exchanger.

12. Method according to one of Claims 1 to 11, characterised in that the byproducts are removed by fractionated precipitation with solvents.

13. Method according to one of Claims 1 to 12, characterised in that the D-glucose is replaced continually to the extent by which it is consumed as an acceptor.

14. Method according to one of Claims 1 to 13, characterised in that anti-mitotic agents (mitosis inhibitors) are added to the reaction mixture in order to avoid unwanted growth of yeasts.

15. Method according to Claim 14, characterised in that sulphurous acid is added in quantities of up to 1000 mg/kg, preferably 400 to 600 mg/kg.

16. Use of a mixture of the completely linear iso-malto-oligosaccharides IM-4 to IM-8, in particular IM-5 to IM-7, obtained according to one of Claims 1 to 15 for preparing a medicament for preventing anaphylactoid side-effects of dextran.

**Revendications**

1. Procédé de production d'haptènes monovalents, caractérisé en ce qu'on ajoute à une solution aqueuse de D-glucose qui contient 400 à 800 mmoles de glucose pour 1000 U d'α(1→6)-D-glucosyltransférase, à 265-310 K et à une valeur de pH de 4,5 à 8,0, une solution aqueuse de saccharose en une quantité telle que le rapport molaire du saccharose au glucose s'élève à une valeur de 0,5 à 2,0, et après la consommation du saccharose, on sépare le glucose, le fructose libéré et les oligosaccharides non désirés d'une manière connue.

2. Procédé suivant la revendication 1, caractérisé en ce qu'on conduit la réaction enzymatique à 290-300 K.

3. Procédé suivant la revendication 1 ou 2, caractérisé en ce que la valeur de pH du mélange réactionnel s'élève à 5-6,5.

4. Procédé suivant l'une des revendications 1 à 3, caractérisé en ce que le rapport molaire du saccharose au glucose s'élève à 0,8-1,2, notamment à 1:1.

5. Procédé suivant l'une des revendications 1 à 4, caractérisé en ce que la solution de saccharose est ajoutée en continu.

6. Procédé suivant l'une des revendications 1 à 5, caractérisé en ce que la solution de saccharose est ajoutée à une vitesse telle que le saccharose soit converti directement par l'α(1→6)-D-glucosyltransférase.

7. Procédé suivant l'une des revendications 1 à 6, caractérisé en ce que l'introduction de saccharose est terminée lorsque la proportion d'iso-malto-oligosaccharides ayant 10 ou plus de 10 motifs anhydroglucose a atteint 10 % des glucides sur base sèche du mélange réactionnel.

8. Procédé suivant l'une des revendications 1 à 7, caractérisé en ce que la teneur en glucides sur base sèche du mélange réactionnel s'élève à 30-50 %, notamment à 40-50 %.

9. Procédé suivant l'une des revendications 1 à 8, caractérisé en ce qu'on utilise comme α(1→6)-D-glucosyltransférase, la dextrane-saccharase élaborée par la bactérie Leuconostoc mesenteroides, notamment la souche B-512.

**10.** Procédé suivant l'une des revendications 1 à 8, caractérisé en ce qu'on utilise comme α(1→6)-D-glucosyltransférase la dextrane-saccharase élaborée par la bactérie Leuconostoc dextranicum.

**11.** Procédé suivant l'une des revendications 1 à 10, caractérisé en ce qu'on effectue la séparation des sous-produits par chromatographie sur une colonne garnie d'un échangeur cationique fortement acide.

**12.** Procédé suivant l'une des revendications 1 à 11, caractérisé en ce qu'on élimine les sous-produits par précipitation fractionnée avec des solvants.

**13.** Procédé suivant l'une des revendications 1 à 12, caractérisé en ce qu'on remplace continuellement le D-glucose au fur et à mesure qu'il est consommé comme accepteur.

**14.** Procédé suivant l'une des revendications 1 à 13, caractérisé en ce qu'on ajoute des agents antimitotiques (inhibiteurs de mitose) au mélange réactionnel pour éviter le développement indésirable d'une levure.

**15.** Procédé suivant la revendication 14, caractérisé en ce qu'on ajoute de l'acide sulfureux en quantités allant jusqu'à 1000 mg/kg, de préférence de 400 à 600 mg/kg.

**16.** Utilisation d'un mélange, obtenu selon l'une des revendications 1 à 15, des isomalto-oligosaccharides entièrement linéaires IM-4 à IM-8, notamment IM-5 à IM-7, pour la préparation d'un médicament destiné à inhiber les effets secondaires anaphylactoïdes du dextrane.